(19) Europäisches Patentamt
European Patent Office
Office européen des brevets

(11) Publication number: **0 001 633**
**A1**

(12) # EUROPEAN PATENT APPLICATION

(21) Application number: 78101169.7

(22) Date of filing: 17.10.78

(51) Int. Cl.[2]: **C 07 D 295/02**
C 07 D 295/14
//C07D239/48, C07D487/04

(30) Priority: 18.10.77 GB 43211/77

(43) Date of publication of application:
02.05.79 Bulletin 79/9

(84) Designated contracting states:
CH DE FR

(71) Applicant: THE WELLCOME FOUNDATION LIMITED
183-193 Euston Road
London NW1 2BP(GB)

(72) Inventor: Swaringen, Roy Archibald, Jnr.
824 Sandiewood Drive
Durham North Carolina 27712(US)

(74) Representative: Berg, Wilhelm, Dr. et al,
Dr. Berg, Dipl.-Ing. Stapf, Dipl.-Ing. Schwabe, Dr. Dr. Sandmair Patentanwälte Postfach 860245
D-8000 München 80(DE)

(54) **Processes for preparing tri-heterocyclic substituted methanes and their conversion into further intermediates useful in the preparation of pharmacologically active compounds.**

(57) The present invention provides a process for preparing useful chemical intermediates, tri-heterocyclic substituted methanes, and their conversion into further intermediates useful in the preparation of pharmacologically active compounds, such as trimethoprim and allopurinol.

EP 0 001 633 A1

This invention relates to a method of preparation of a one-carbon reagent suitable for use in the synthesis of pharmaceuticals. Most particularly, the present invention relates to a method of preparation of certain tri-*sec*-aminomethanes and their use in the preparation of intermediates suitable for the synthesis of pharmaceuticals.

A number of methods of synthesis of tri-*sec*-aminomethanes are known from the literature.

Scheeren and Nivard, *Rec. Trav. Chim.*, 1969, *88*, 289 disclosed two methods for the preparation of tri-*sec*-aminomethanes. According to their first method, the compounds may be synthesised from chloroform, methanolic sodium methoxide and the appropriate secondary amine. The yield by this method for trimorpholinomethane is of the order of 47% with an additional 31% obtainable by reprocessing the filtrate with morpholine, but although this is quite a reasonable yield, the process requires strictly anhydrous conditions and is somewhat complicated for large scale work. For the other tri-*sec*-aminomethanes reported therein, the yield is considerably lower. Scheeren and Nivard's

second disclosed method describes the preparation of tri-*sec*-aminomethanes from the appropriate secondary amine and dimethylformamide dimethyl acetal. Although the yield was 75% when the secondary amine was morpholine, the very high cost of dimethylformamide dimethyl-acetal inhibits the application of this synthesis on a commercial scale. Again, for a number of the other tri-*sec*-aminomethanes reported therein, the yield is considerably lower.

Bredereck *et. al. Liebigs Ann. Chem.*, 1972, *762*, 62 describe the reaction of the adduct of N-formylmorpholine and dimethyl sulphate with excess morpholine to form dimorpholinoformamidinium methyl sulphate, with a subsequent reaction with potassium methylate. An equilibrium exists in this latter reaction which is driven towards trimorpholinomethane by the fact that it precipitates. This synthesis, however, is difficult due to hygroscopic intermediates and heterogeneous reaction mixtures and furthermore, the yield of trimorpholinomethane, based on N-formylmorpholine as starting material is only 21%.

It has now been found that tri-sec-aminomethanes may be prepared by a method that is convenient, economical and high yielding.

According to one aspect of the present invention, therefore, there is provided a process for preparing a tri-sec-aminomethane of the formula:

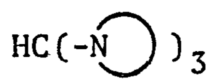

wherein 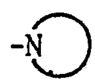 forms a morpholino, piperidino or an N-methylpiperazino group, which process comprises the reaction of an orthoformate of the formula (I):

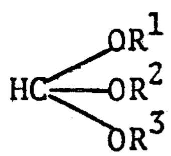

(I)

wherein $R^1$, $R^2$ and $R^3$ are the same or different and each is a $C_{1-4}$ alkyl group, with the appropriate six-membered saturated heterocycle 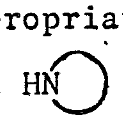 in the presence of an acid or acid salt catalyst, under conditions which allow the alkanol formed to be continuously removed.

The above reaction to produce the tri-sec-aminomethanes is a reaction leading to an equilibrium and thus in order for it to proceed to completion, the removal of the alkanol side products is necessary. In order to accomplish this, the reaction may, for instance, be run under reflux, removal of the side products being effected either by use of a heated, e.g. steam-jacketed reflux column, or by use of a normal reflux column in combination with molecular sieves for scavenging the unwanted alkanols or by removing the side product by azeotropy with a suitable solvent. The first method has the slight disadvantage that higher temperatures in the reaction medium are involved.

With regard to the orthoformates employed in the process of the present invention, a general requirement when using a steam-jacketed column is that an orthoformate having a boiling point greater than 100°C, derived from an alkanol boiling below 100°C, is suitable to achieve appropriate separation of the latter in the reaction. Mixed orthoformates may also be used provided they fulfill the above requirement,

but they are generally less available commercially than those where all three alkoxy groups are the same, and for the purposes of the present invention, triethyl orthoformate is the most preferred orthoformate.

The molar ratio of heterocycle 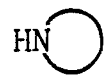 to orthoformate in the process of the present invention is conveniently in the range of 1:2 to 4:1, preferably in the range of 1:1 to 3:1, and most preferably in the ratio of 2:1.

Catalysts for use in the present invention may be any acid or acid salt which is stable at the reaction temperature. Preferably they are anhydrous and examples of acids and acid salts which may be employed for the purposes of the present invention as catalysts are: benzoic acids, sulphuric acid, $NaHSO_4$, $KaH_2PO_4$, pivalic acid, p-toluenesulphonic acid and most preferably glacial acetic acid. More than one catalyst may be employed at any one time although it is preferable to use only a single catalyst.

The concentration of catalyst required for the process of the present invention is conveniently in the range of 0.5 to 20 mole

per cent based on theoretical yield of the tri-*sec*-aminomethane and preferably in the range of 1 to 15 mole per cent based on the theoretical yield of the tri-*sec*-aminomethane. It should be noted that too much acid or acid salt catalyst could cause the formation of a complex with the secondary amine or else consume the orthoformate employed.

Although no solvent is required for the process of the present invention, it has been found that solvents may be used with advantage if so desired. Suitable solvents are those which are inert to orthoformates, to the amine and to the combination. Thus, it is preferred to use solvents which are not protic or esters and do not contain acidic C-H, $CH_2$ or $CH_3$ groups. In addition, the boiling point of the solvent must be above 100°C if a steam-jacketed column is used. The most suitable solvents are aromatic solvents such as benzene, toluene and xylene and also ethers such as dioxane and diglyme. Benzene and toluene are particularly preferred since the alkanol by-product may be removed as an azeotrope.

The tri-*sec*-aminomethanes of the present invention have been found to be useful one-carbon reagents, and may be used in the preparation of intermediates, such as 3-amino-pyrazole-4-carboxamide hemisulphate, α-(3,4,5-trimethoxybenzyl)-β-piperidino-acrylonitrile and α-(3,4,5-trimethoxybenzyl)-β-morpholino-acrylonitrile, which are suitable for the synthesis of pharmaceuticals, and in particular for the synthesis of pyrazolo[3,4-*d*]pyrimidines and 2,4-diamino-5-benzylpyrimidines.

4-Hydroxypyrazolo[3,4-*d*]pyrimidine, which is also known as allopurinol, is active as an inhibitor of the enzyme xanthine oxidase which catalyses the oxidation of most purines to uric acid *in vivo*. Allopurinol has thus been found to relieve the symptoms of gout since it greatly diminishes the amount of uric acid formed. In the same manner, it slows down the oxidation of 6-mercaptopurine to 6-thiouric acid, thus permitting smaller doses of 6-mercaptopurine to be employed.

Allopurinol is administered to patients in relatively large doses, generally in the range

of 100 to 800 mg per person per day, but sometimes in still larger doses. Also, allopurinol is given over protracted periods.

The purity of the product is thus of even greater importance than with most pharmaceuticals. Some contaminants have been found difficult and expensive to remove from the final product and it is consequently of great practical importance that a method of synthesis should not only be quantitatively efficient, but also that the final product should be extremely pure.

Examples of other pyrazolo[3,4-d]pyrimidines possessing valuable xanthine oxidase inhibiting activity are 4,6-dihydroxypyrazolo[3,4-d]pyrimidine, also known as oxypurinol, and 4-mercaptopyrazolo-[3,4-d]pyrimidine, also known as thiopurinol.

A number of methods of synthesis are known for pyrazolo[3,4-d]pyrimidines such as those described above. For example, allopurinol may be prepared by reacting ethoxymethylenemalononitrile with hydrazine to give 3-amino-4-cyanopyrazole, hydrolysing the 3-amino-4-cyanopyrazole to 3-aminopyrazole-4-carboxamide,

and reacting the 3-aminopyrazole-4-carboxamide with formamide and/or formic acid. Oxypurinol may be prepared by a similar synthetic route in which urea is used instead of formamide and/or formic acid. A more recent method of synthesis is that which is described in British Patent Specification No. 1 252 435. In this method, a compound of formula (II):

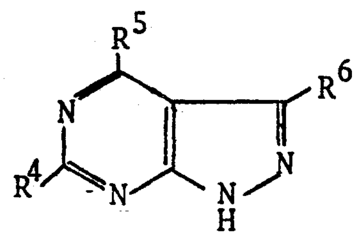

(II)

wherein $R^6$ is a hydrogen atom or an alkyl group having 1 to 4 carbon atoms, $R^5$ is a hydroxy group or a mercapto group, and $R^4$ is a hydrogen atom or a hydroxy group, provided that $R^4$ is not a hydroxy group when $R^5$ is a mercapto group, is prepared by reacting a compound of formula (III):

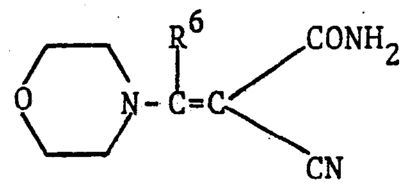

(III)

with hydrazine to produce a compound of formula (IV):

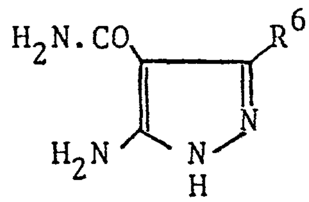

(IV)

and reacting a compound of formula (IV) with urea or with formamide and/or formic acid to produce a compound of formula (II).

The reaction of a compound of formula (III) with hydrazine is conveniently carried out in the presence of heat. The compound of formula (IV) produced by the reaction is a base but, if it is to be isolated, it is preferably converted by reaction with an acid into a salt, desirably a salt of a pharmaceutically acceptable acid such as sulphuric acid.

A compound of formula (II) so produced by the complete sequence can be converted to a different compound of formula (II) by standard methods. For example, 4-hydroxy-pyrazolo-[3,4-d]pyrimidine can be converted to 4-mercaptopyrazolo[3,4-d]pyrimidine by reaction

with a sulphide such as phosphorus pentasulphide.

The preparation of compounds of formula (III) is described in British Patent Specification No. 1 252 436. In this method, the morpholinocyanoacrylamides are prepared by reacting a compound $R^6C(OR^7)_3$, wherein $R^7$ is an alkyl group having from 1 to 4 carbon atoms, and is preferably an ethyl group, and $R^6$ is as hereinabove defined, with cyanoacetamide and morpholino under reflux.

It has now been found that the compound of formula (III) wherein $R^6$ is a hydrogen atom can be prepared more conveniently and in higher yield than by that described in the aforementioned Patent Specification by reacting cyanoacetamide with trimorpholinomethane. The 3-piperidino and 3-N-methylpiperazino analogues of the compound of the formula (III) wherein $R^6$ is a hydrogen atom can also be prepared according to the present invention by reacting cyanoacetamide with tripiperidinomethane and tri-N-methylpiperazinomethane respectively.

These compounds can then be converted into a compound of the formula (IV) wherein $R^6$ is a hydrogen atom by the reaction with hydrazine.

Accordingly in a further aspect, the present invention provides a process for the preparation of a compound of the formula (V):

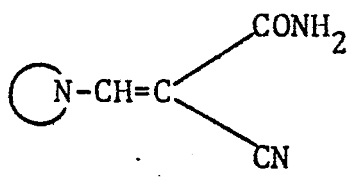

(V)

wherein 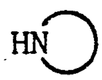 is morpholine, piperidine or N-methylpiperazine, which process comprises the reaction of cyanoacetamide with a tri-<u>sec</u>-aminomethane of the formula HC(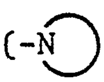)$_3$ as hereinbefore defined.

Preferably equimolar quantities or a slight excess of trimorpholinomethane is employed in the reaction, which may preferably be carried out in the presence of an alkanol, for example ethanol, as solvent and at a temperature in the range of 20 to 100°C, preferably 35 to 65°C, most preferably 55°C. The reaction is simple and convenient to perform, the reactants dissolving readily in the reaction medium, and proceeds to

completion in a relatively short time without the necessity of refluxing. Furthermore, the solvent and the morpholine by-product are easily removed *in vacuo* to give a high yield pure product. Samples of pure allopurinol and related compounds may be synthesised from 3-morpholino-2-cyanoacrylamide which has been prepared by the method of the present invention.

2,4-Diamino-5-benzylpyrimidines possess both antimalarial and antibacterial activities (*J. Chem. Soc.* (1951), *73*, 37-58). Maximal antibacterial activity is found among derivatives which bear electron donating substituents in the benzene nucleus and are unsubstituted in the 6th position of the pyrimidine moiety. 2,4-Diamino-5-(3',4',5'-trimethoxybenzyl)pyrimidine or trimethoprim (U.S. Patent No. 2 909 522) has a moderately broad antibacterial spectrum which includes many of the Gram-positive species but is also active against species of the genus *Proteus*. In common with other 2,4-diaminopyrimidines it is a competitor of folic and folinic acids in microorganisms which require

these nutrients and it can be shown to inhibit dihydrofolate reductase in S. faecalis. A strong potentiative effect is observed when the drug is administered in combination with sulphonamides as a consequence of the sequential blockade of the biochemical pathway which leads to the de novo synthesis of coenzyme F. 2,4-diamino-5-benzylpyrimidines, which include trimethoprim and 2,4-diamino-5-(3',4'-dimethoxybenzyl)pyrimidine or diaveridine (U.S. Patent No. 2 658 897), may be administered orally at a dose of 1 mg/kg to 30 mg/kg per day.

A number of methods of synthesis are known for 2,4-diamino-benzylpyrimidines such as those described above. For example, they may be prepared by the method of Stenbuck et. al. (1963), 28, 1983 (British Patent Specification No. 957 797). This route comprises the steps of condensing an aromatic aldehyde with a β-substituted propionitrile in the presence of both an alkanol as solvent and a strong base to give a mixture of isomeric intermediates, and reacting these intermediates with guanidine to give a 5-benzylpyrimidine.

A more recent method of synthesis is that which is described in British Patent Specification No. 1 261 455. In this method, a compound of formula (VI):

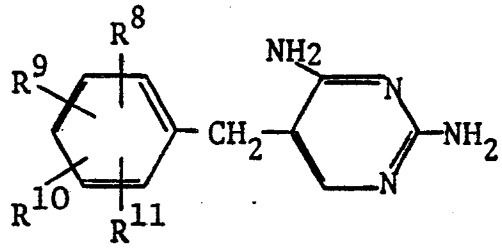

(VI)

wherein $R^8$, $R^9$, $R^{10}$ and $R^{11}$ are the same or different and each is a hydrogen or a halogen atom, an alkyl, alkoxy or benzyloxy group, or $R^{10}$ and $R^{11}$ taken together may be a methylenedioxy group is prepared by reacting a compound of formula (VII), substantially free from contamination with the β-amino-α-benzylidenepropionitrile isomer,

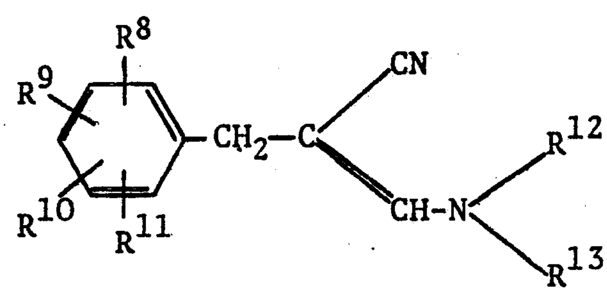

(VII)

wherein $R^8$, $R^9$, $R^{10}$ and $R^{11}$ are as hereinbefore defined and $NR^{12}R^{13}$ is as hereinbelow

defined, with guanidine.

The β-amino group $NR^{12}R^{13}$ is an aliphatic, heterocyclic or aromatic group, and can only have one hydrogen for $R^{12}$ and $R^{13}$, and the above reaction is preferably performed at reflux temperature.

The compounds of formula (VII) are prepared by reacting the corresponding benzaldehyde with the corresponding β-amino-propionitrile in the presence of a base in a polar aprotic solvent compatibel with and dissolving both reactants.

It has now been found that the compounds of formula (VI) wherein the $NR^{12}R^{13}$ moiety is a group 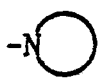 as defined herein may be prepared conveniently by reacting the corresponding β-phenylpropionitrile with the appropriate tri-sec-aminomethane.

Thus, according to a further aspect of the present invention there is provided a process for the preparation of a compound of formula (VIII):

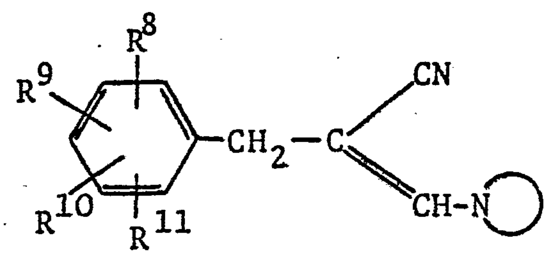

(VIII)

wherein $R^8$, $R^9$, $R^{10}$ and $R^{11}$ are as hereinbefore defined which comprises reacting a compound of formula (IX):

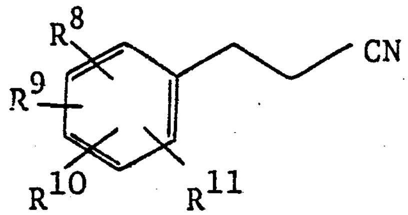

(IX)

wherein $R^8$, $R^9$, $R^{10}$ and $R^{11}$ are as hereinbefore defined with a tri-sec-aminomethane of the formula HC(-N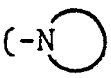)$_3$ as defined herein.

Preferably equimolar quantities of reactants or a slight excess of the tri-sec-aminomethane is employed in the reaction which is carried out without a solvent, in which case the compound of the formula (IX) is preferably first melted before addition of the tri-sec-aminomethane. The reaction is carried out under an inert gas atmosphere, for example nitrogen or argon, or under vacuum at a temperature of 110° to 190°C, preferably 130° to 170°C, most preferably 150°C. The reaction is simple and convenient to perform and proceeds to completion in a relatively short time, for example to 4 hours.

The following Examples illustrate the invention but are not intended to limit it in any way.

EXAMPLE 1

Trimorpholinomethane

Morpholine (87.1 g), triethyl orthoformate (74.1 g), and acetic acid (3.0 g) were combined and heated for 2 hours at reflux using a steam-jacketed column for continuous removal of ethanol. The residual solution in the reaction vessel, having reached a temperature of 180°C, was allowed to cool so that the product could crystallise out. The crystals were filtered, washed with ether, and immediately dried *in vacuo*, due to the hygroscopic character of the crystals to yield trimorpholinomethane (57.1 g; 63.1% of theoretical yield; m.p. 147-151°C, n.m.r. ($CDCl_3$) $\delta$ 1.42 (*t*, 12, $-CH_2N$), 3.27; (s, 1, $HC(N)_3$), and 3.65 (*t*, 12, $-CH_2O$).

EXAMPLE 2

Trimorpholinomethane

The procedure of Example 1 was followed with the exception that *p*-toluenesulphonic acid (1.0 g) was used instead of acetic acid as catalyst. Trimorpholinomethane (51.2 g; 56% of theoretical yield) was obtained.

EXAMPLE 3

3-Morpholino-2-cyanoacrylamide

A slurry of trimorpholinomethane (29.9 g) in ethanol (100 ml) was added to a solution of cyanoacetamide (8.4 g) in ethanol (150 ml) at 55°C. All material dissolved rapidly. After 30 minutes the ethanol and the morpholine by-product were removed *in vacuo*, and the residue was washed with ether and dried to yield 3-morpholino-2-cyanoacrylamide (16.4 g; 91% of theoretical yield m.p. 170-174°C.

EXAMPLE 4

Tri-(N'-methylpiperazino)methane

*N*-methylpiperazine (100.2 g, 1.0 mol), triethyl orthoformate (74.1 g, 0.5 mol), and acetic acid (3.0 g, 0.05 mol) were combined and heated to reflux using a steam-jacketed column for continuous removal of ethanol. After 2 hours the pot temperature had risen to ∿ 180° (from ∿ 130° initially). The reaction mixture was cooled and subjected to vacuum distillation (0.1 mmHg) to remove unreacted starting materials and some N-formyl-N'-methylpiperazine. Upon cooling the pot residue solidified. The solid was triturated

with 100 ml of ether and filtered to yield 40.7 g (39.3%) of tri-(N'-methylpiperazino)methane. Concentration of the ether wash gave a second crop, 32.4 g (31.3%). NMR ($CDCl_3$) δ 2.25 (s, 9, $CH_3N$), 2.3 (t, 12, piperazine ing H), 3.07 (t, 12, piperazine ring H), and 3.3 (s, 1, $HC(N)_3$).

EXAMPLE 5

Tripiperidinomethane

Piperidine (153.3 g, 1.80 mol), triethyl orthoformate (133.4 g, 0.90 mol), and acetic acid (3.6 g, 0.06 mol) were combined in a 500 ml three-neck round bottom flask equipped with a thermometer and a steam-jacketed condenser. The reactants were heated to a gentle reflux (initial pot temperature ~ 112°). The heat input was gradually increased during 29.5 hours of reflux; the final pot temperature was 144°C. The reaction solution was allowed to cool and stand overnight and then was concentrated *in vacuo*. High vacuum distillation of the crude product gave 19.3 g of N-formylpiperidine (b.p. ~ 60-70°/0.1-0.2 mmHg) and 69.6 g (43.7% yield) of tripiperidinomethane (b.p. 98-106°/0.05-0.1 mmHg).

EXAMPLE 6

Tripiperidinomethane

Piperidine (170.3 g, 2.0 mol), triethyl orthoformate (74.1 g, 0.50 mol), and acetic acid (3.0 g, 0.05 mol) were refluxed together for 22 hours using a steam-jacketed condenser packed with glass helices. The mixture was then placed on a rotary evaporator to remove unreacted piperidine and triethyl orthoformate, and then subjected to vacuum distillation to remove the by-product N-formylpiperidine. The residue of tripiperidinomethane (65.6 g, 49%) crystallised upon standing, m.p. 42-48$^{o}$C. NMR ($CDCl_3$): 1.41 (s, 18, $-(CH_2)_3-$), 2.60 (s, 12, $N-(CH_2)_2$), 3.14 (s, 1, $CH(N)_3$).

EXAMPLE 7

Trimorpholinomethane

Morpholine (52.3 g, 0.60 mol), trimethyl orthoformate (42.5 g, 0.40 mol), and acetic acid (1.2 g, 0.02 mol) were refluxed together for 24 hours using a Soxhlet extractor filled with 100 g of 4A molecular sieves. Upon cooling the mixture solidified. The solid was triturated with ether,

filtered, washed, and dried overnight in a vacuum dessicator to give 24.1 g (45%) of trimorpholinomethane.

EXAMPLE 8

Trimorpholinomethane

Morpholine (26.1 g, 0.30 mol), triethyl orthoformate (29.6 g, 0.20 mol), pivalic acid (1.0 g, 0.01 mol), and toluene (500 mls) were heated to boiling in a distillation apparatus. Continuous azeotropic distillation of toluene removed the ethanol by-product. During the first four hours, 325 mls of distillate were collected. Fresh toluene (250 mls) was added and the distillation continued for another two hours with an additional 260 mls collected. Upon cooling, the crystalline product was removed by filtration (8.5 g, 31%). Evaporation of solvent and trituration of the residue with ether gave a second crop (3.4 g, 13%). Concentration of the mother liquor yielded a third crop (5.0 g, 18%). The total yield was 16.9 g (62%).

EXAMPLES 9-19

Using methods strictly analogous to those described in Examples 1-8 herein ortho amides were prepared from the following starting materials in stated yields under the following conditions:-

| AMINE (moles) | ORTHOFORMATE (moles) | ACID (moles) | FINAL TEMP. °C | TIME hours | % YIELD ortho amide | METHC |
|---|---|---|---|---|---|---|
| Piperidine (2.0) | Triethyl (0.50) | Aceti: (0.05) | 158 | 22 | 49 | d |
| Piperidine (0.64) | Triisopropyl (0.16) | Acetic (0.016) | 123 | 22 | 62 | d |
| Piperidine (2.0) | Triethyl (0.50) | Pivalic (0.05) | 140 | 23 | 51 | d |
| Morpholine (0.60) | Triethyl (0.40) | Pivalic (0.02) | 170 | 1.5 | 66 | a |
| Morpholine (0.60) | Triethyl (0.40) | $KH_2PO_4$ (0.02) | 170 | 21.5 | 57 | a |
| Morpholine (0.60) | Triethyl (0.40) | 4-Chloro-benzoic (0.02) | 156 | 22 | 52 | a |
| Morpholine (0.60) | Trimethyl (0.40) | Acetic (0.02) | 142 | 24 | 45 | f |
| Morpholine (0.30) | Triethyl (0.20) | Pivalic (0.01) | 114 | 6 | 62 | e |
| 2,6-dimethyl-morpholine (1.20) | Triethyl (0.80) | Acetic (0.04) | 142 | 4. | 52 | c* |
| Morpholine (1.0) | Triethyl (0.50) | p-toluene-sulfonic (0.005) | 165 | 18 | 57 | a |
| Morpholine (1.0 | Triethyl (0.50) | Acetic (0.05) | 180 | 2 | 63 | a |

a) Straight-bore steam-jacketed condenser. Crystalline product filtered from reaction mixture.

b) straight-bore steam-jacketed condenser. Product distilled under vacuum.

c) Straight-bore steam-jacketed condenser. Product isolated as pot residue after vacuum distillation.

d) Steam-jacketed condenser packed with glass helices. Product isolated as pot residue after vacuum distillation.

e) Continuous azeotropic distillation of toluene solvent. Crystalline product filtered from reaction mixture.

f) Soxhlet extractor filled with molecular sieves. Crystalline product filtered from reaction mixture.

* Product contained 13% of the ethyl ester aminal.

<u>EXAMPLE 20</u>

<u>α-(3,4,5-Trimethoxybenzyl)-β-morpholinoacrylonitrile</u>

β-(3,4,5-Trimethoxyphenyl)propionitrile (4.40 g) prepared from 3,4,5-trimethoxybenzaldehyde

and cyanoacetic acid followed by catalytic hydrogenation, was melted in a flask equipped with a mechanical stirrer, thermometer, and gas inlet tube. Trimorpholinomethane (5.4 g) was added, and a nitrogen atmosphere was established. The temperature was raised to 150°C and maintained for 3 hours. After cooling to room temperature the solid was washed thoroughly with ether and filtered. The crude product was purified by treatment with a warm mixture of chloroform/hexane (1/1) and filtration to remove a small amount of insolubles. Evaporation of the solvent gave α-(3,4,5-trimethoxybenzyl)-β-morpholino-acrylonitrile (2.4 g; 38% of theoretical yield). M.P. 105-107°C.

EXAMPLE 21

3-Aminopyrazole-4-carboxamide hemi-sulphate

To water (253 ml) at 60°C was added 3-morpholino-2-cyanoacrylamide (63.4 g) and 85% of technical hydrazine hydrate (22.7 g). The mixture was rapidly heated to 95°C and the temperature was maintained at > 90°C for 20 minutes. The mixture was then cooled to 60°C and the pH carefully adjusted to 1.5 by the addition of a mixture

of sulphuric acid (45.7 g) and ice (45.7 g). The acidified reaction mixture was cooled to 5°C and the crystalline product collected and washed with cold water (2 x 100 ml) and acetone (2 x 50 ml). The product was dried *in vacuo* at 80°C. Wt. = 5.8 g. Yield = 95%. M.P. 237-239°C.

EXAMPLE 22

4-Hydroxypyrazolo(3,4-d)pyrimidine(allopurinol)

A suspension of 3-aminopyrazole-4-carboxamide hemisulphate (113 g) in formamide (325 ml) was stirred and heated to 145°C. The reaction was then cooled to 30°C and the product collected and washed with formamide (2 x 50 ml), water (2 x 150 ml) and acetone (2 x 100 ml). Wt. of crude product = 79 g. The crude product was recrystallised by dissolution in a solution made from sodium hydroxide (25 g) in water (1200 ml) with treatment at 25°C with charcoal (8 g) followed by reprecipitation by the addition of concentrated hydrochloric acid to pH 5. The product was collected and washed with cold water (2 x 300 ml), acetone (2 x 200 ml) and dried *in vacuo* at 60°C. Wt. = 70 g. Yield 80%.

EXAMPLE 23

2,4-Diamino-5-(3',4',5'-trimethoxybenzyl)pyrimidine (trimethoprim)

α-(3,4,5-Trimethoxybenzyl)-β-morpholino-acrylonitrile (32.0 g), guanidine carbonate (34.0 g) and dimethylsulphoxide (50 ml) were heated together at 160°C for 1 hour with stirring. The reaction mixture was cooled and poured into ice-water (200 ml), and gave 2,4-diamino-5-(3',4',5-trimethoxybenzyl) pyrimidine which was collected and washed with water and acetone. Wt. = 23.6 g (80% theoretical yield). M.P. 196-198°C.

EXAMPLE 24

α-(3,4,5-Trimethoxybenzyl)-β-piperidinoacrylonitrile

Tripiperidinomethane (1.86 g, 7 mmol) and 3-(3,4,5-trimethoxyphenyl)propionitrile (1.11 g, 5 mmol) were combined quickly and heated for 18 hours at 135°C (pot temperature) under house vacuum (125 mmHg). The resultant brown oil was taken up in ether (3 ml) and placed on a short silica gel column. The column was washed with dichloromethane and the washings concentrated to an oil which solidified on standing. The product was washed with

ether (3 x 10 ml) and dried to yield 0.88 g (55.5% of a beige solid (m.p. 100-101.5°C), whose nmr spectrum was consistent with the desired structure. A second crop of light yellow solid (m.p. 86-92°C) was isolated from the combined ether washings. NMR analysis showed the second crop to be an 85:15 mixture of expected product and starting nitrile. The combined assay yield for the two crops was 1.075 g (68.0%).

EXAMPLE 25

2-Cyano-3-piperidinoacrylamide

Tripiperidinomethane (58.4 g, 0.22 mol) and cyanoacetamide (16.8 g, 0.20 mol) were combined in 200 mls of ethanol and stirred for 4.5 hours at room temperature. After cooling, the crystals were filtered, washed, and dried to give 25.3 g (71%) of 2-cyano-3-piperidinoacrylamide, m.p. 159-161°C. NMR ($CDCl_3$): δ 1.70 (ss 6, $-(CH_2)_3-$), δ 3.50 and 3.90 (two broadened singlets, 4, $N-(CH_2)_2$), δ 5.95 (broad s, exchange with $D_2O$, 2, $-NH_2$), δ 7.90 (s, 1, CH-). Elemental analysis, calculated for $C_9H_{13}N_3O$: C,60.32; H,7.31; N,23.45%. Found: C,60.39; H,7.39; N,23.53%.

EXAMPLE 26

3-Aminopyrazole-4-carboxamide hemisulfate

To a solution of 2-cyano-3-piperidinoacrylamide (7.90 g, 0.044 mol) in 75 mls of ethanol at 66°C was added a solution of 85% hydrazine hydrate (2.86 g, 0.048 mol) in 10 mls of ethanol. After refluxing for three hours, the mixture was cooled to 50°C and a solution of concentrated $H_2SO_4$ (6.46 g, 0.066 mol) in 20 mls of ethanol was added. After cooling, the product was filtered, washed, and dried to give 6.16 g (80%) of 3-aminopyrazole-4-carboxamide hemisulfate, 91% pure by uv analysis.

WHAT WE CLAIM

1. A process for preparing a tri-sec-amino-methane of the formula:

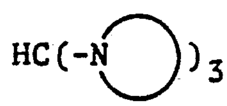

wherein 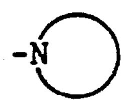 forms a morpholino, piperidino or N-methylpiperazino group, which comprises the reaction of an orthoformate of the fomula (I):

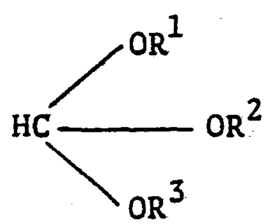 (I)

wherein $R^1$, $R^2$ and $R^3$ are the same or different and each is a $C_{1-4}$ alkyl group, with the appropriate six-membered saturated heterocycle 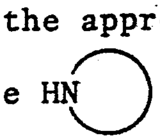 in the presence of an acid or an acid salt catalyst, under

conditions which allow the alkanol formed to be continuously removed.

2. A process according to claim 1 wherein the orthoformate of the formula (I) is triethyl orthoformate.

3. A process according to either claim 1 or 2 wherein the molar ratio of the heterocycle 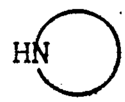 to orthoformate is 2:1.

4. A process according to any one of claims 1-5 wherein the acid or acid salt is selected from benzoic acids, sulphuric acid, $NaHSO_4$, $KaH_2PO_4$, p-toluenesulphonic acid, pivalic acid and glacial acetic acid.

5. A process according to any one of claims 1-4 wherein the acid or acid salt is present in the range of 0.5 to 20 mole per cent based on the theoretical yield of the tri-<u>sec</u>-aminomethane.

6. A process according to any one of claims 1-5 wherein the reaction is carried out in the presence of a suitable solvent as hereinbefore defined.

7. A process for the preparation of a compound of the formula (V):

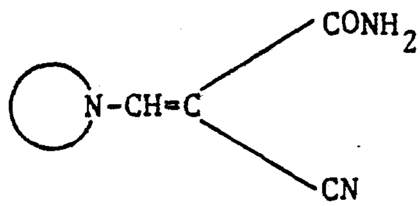

(V)

wherein 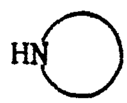 is morpholine, piperidine or N-methylpiperazine, which comprises the reaction of cyanoacetamide with a tri-sec-aminomethane of the formula HC(- 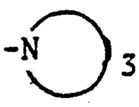 as hereinbefore defined.

8. A process for the preparation of a compound of the formula (VIII):

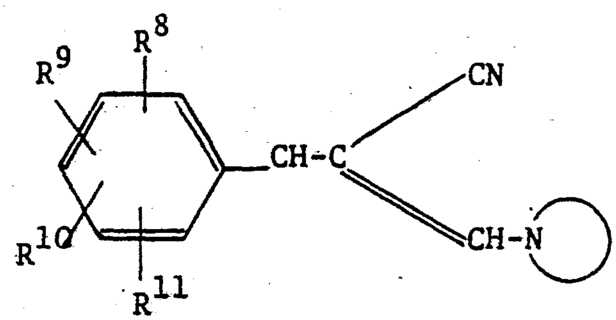

wherein $R^8$, $R^9$, $R^{10}$ and $R^{11}$ are the same or different and each is a hydrogen or a halogen atom, an alkyl, alkoxy or benzyloxy group, or $R^{10}$ and $R^{11}$ taken together may be a methylenedioxy group and 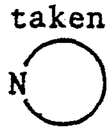, is a morpholino, piperidino or N-methylpiperazino group, which comprises the reaction of a compound of the formula (IX):

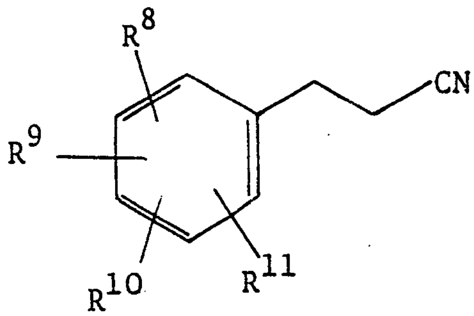

(IX)

wherein $R^8$, $R^9$, $R^{10}$ and $R^{11}$ are as hereinbefore defined with a tri-sec-aminomethane of the formula HC(-N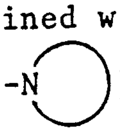)$_3$ as hereinbefore defined.

9. A process according to claim 8 wherein equimolar quantities of the reactants or a slight excess of the tri-sec-aminomethane is employed.

0001633

European Patent Office

Application number

EP 78 101 169.7

# EUROPEAN SEARCH REPORT

| DOCUMENTS CONSIDERED TO BE RELEVANT | | | CLASSIFICATION OF THE APPLICATION (Int. Cl.²) |
|---|---|---|---|
| Category | Citation of document with indication, where appropriate, of relevant passages | Relevant to claim | |
| - | Chemical Abstracts vol. 75, no. 19, 8 November 1971, Columbus, Ohio, USA O. TSUGE et al "Tetraminoethylenes I. Preparation and some reactions of tetrapiperidino- and tetramorpholinoethylene" page 216, column 2, abstract no. 118 277 x & Bull. Chem. Soc. Jap. 1971, 44(8) pages 2171-6 | 1 | C 07 D 295/02<br>C 07 D 295/14//<br>C 07 D 239/48<br>C 07 D 487/04 |
| - | HOUBEN-WEYL "Methoden der organischen Chemie", 4. Auflage, Band VI/3, 1965, GEORG THIEME VERLAG, Stuttgart, pages 317 to 318 | 1 | TECHNICAL FIELDS SEARCHED (Int.Cl.²)<br>C 07 D 211/14<br>C 07 D 241/04<br>C 07 D 265/30<br>C 07 D 295/02<br>C 07 D 295/14 |
| - | DE - A - 1 921 676 (WELLCOME) * page 3 * & GB - A - 1 252 436 | 1,7 | |
| - | DE - A - 2 010 166 (WELLCOME) * claim 65 * | 8 | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| - | GB - A - 1 484 482 (HOFFMANN-LA ROCHE) * page 2, column 1 * | 8 | &: member of the same patent family, corresponding document |

X The present search report has been drawn up for all claims

| Place of search | Date of completion of the search | Examiner |
|---|---|---|
| Berlin | 22-01-1979 | FROELICH |